# EUROPEAN PATENT APPLICATION

(11) **EP 4 628 161 A1**
(43) Date of publication of application: **08.10.2025**
(21) Application number: 23903997.7
(22) Date of filing: 13.12.2023
(51) Int. Cl.: A61N 7/02, A61B 18/04, A61B 18/00

(54) **WATER SUPPLY SYSTEM FOR ULTRASONIC WAVE GENERATION DEVICE**

(30) Priority: 13.12.2022 KR 20220173375; 12.12.2023 KR 20230179422
(71) Applicant: Godius Co., Ltd., Seoul 08501 (KR)
(72) Inventor: KANG, Dong Hwan, Seoul 08501 (KR); KIM, Sun, Seoul 08501 (KR); LEE, Hyun Sook, Seoul 08501 (KR); KIM, Young Joo, Seoul 08501 (KR); KIM, Namkuk, Seoul 08501 (KR)
(74) Representative: dompatent von Kreisler Selting Werner - Partnerschaft von Patent- und Rechtsanwälten mbB
(86) International application number: PCT/KR2023/020541
(87) International publication number: WO 2024/128796

(57) **Abstract**

A water supply system for an ultrasonic generator according to the present disclosure may include an ultrasonic generator including a water membrane for transmitting ultrasonic vibration to a skin; a water supply device configured to supply water to the ultrasonic generator; and a controller configured to selectively control a flow path of the water.

## Description

### [TECHNICAL FIELD]

The present disclosure relates to a water supply system for an ultrasonic generator.

### [BACKGROUND ART]

Ultrasound refers to waves with a frequency of 20 kHz or higher, and has the property of penetrating water, so it is widely used in the medical field, such as ultrasound diagnostic devices and ultrasound treatment devices.

The most representative application of ultrasound in the medical field is an ultrasound imaging device that utilizes the properties of ultrasound penetration and reflection. For example, there is a device that visualizes the time and intensity of reflection as ultrasound penetrates the human body and passes through each organ, thereby obtaining a cross-sectional image of the human body.

In addition, there is a device that uses the heat generated by high-intensity focused ultrasound (HIFU) to burn and remove specific subcutaneous tissues, such as tumors in the skin, or to induce degeneration and regeneration of skin tissue, resulting in skin beauty or skin plastic surgery effects, such as wrinkle improvement.

However, the conventional system for supplying water to an ultrasonic generator had limitations in shortening the treatment preparation time, had limitations in suppressing the temperature rise of the ultrasonic generator during the treatment, and had limitations in improving the degassing and cooling efficiency.

### [DETAILED DESCRIPTION OF THE INVENTION]

### [TECHINICAL PROBLEM]

The purpose of the embodiment disclosed in the present disclosure is to shorten a procedure preparation time and suppress a temperature rise of an ultrasonic generator during the procedure.

In addition, the purpose of the embodiment disclosed in the present invention is to improve a degassing and cooling efficiency.

Technical problems of the inventive concept are not limited to the technical problems mentioned above, and other technical problems not mentioned will be clearly understood by those skilled in the art from the following description.

### [TECHNICAL SOLUTION]

In an aspect of the present disclosure, a water supply system for an ultrasonic generator may include an ultrasonic generator including a water membrane for transmitting ultrasonic vibration to a skin; a water supply device configured to supply water to the ultrasonic generator; and a controller configured to selectively control a flow path of the water.

Furthermore, the water supply device may include: a supply part for discharging water from the water membrane and supplying the discharged water to the water membrane; a cooling part for cooling the water supplied to the water membrane; a degassing part for removing dissolved gas in the cooled water; and a valve connected to the water membrane, the supply part, the cooling part, and the degassing part.

Furthermore, the valve may include a first valve, a second valve, and a third valve, and the third valve may be connected to the water membrane and the supply part, the first valve may be connected to the third valve and the supply part, and the second valve may be connected to the degassing part, the supply part, and the water membrane.

Furthermore, the supply part may include: a discharge pump for pumping water discharged from the water membrane; a water tank for storing the water pumped by the discharge pump; a supply pump for pumping the water discharged from the water tank and supplies the water to the water membrane; and a water purification part for purifying the water pumped by the supply pump.

Furthermore, the controller may be configured to: open the first valve and the second valve and close the third valve to form a flow path of the water in a direction of the water tank, the supply pump, the water purification part, the cooling part, the degassing part, and the water membrane.

Furthermore, the controller may be configured to: open the first valve and the second valve and close the third valve to form a flow path of the water in a direction of the water tank, the supply pump, the water purification part, the cooling part, the degassing part, and the water tank.

Furthermore, the controller may be configured to: open the first valve, the second valve, and the third valve to form a flow path of the water in a direction of the water membrane, the supply pump, the water purification part, the cooling part, the degassing part, and the water membrane.

Furthermore, the controller may be configured to: open the first valve, the second valve, and the third valve to form a flow path of the water in a direction of the water membrane, the discharge pump, the water tank, the supply pump, the water purifier, the cooling part, the degassing part, and the water membrane.

Furthermore, the controller may be configured to: close the first valve and the second valve, and open the third valve to form a flow path of the water in a direction of the water membrane and the discharge pump.

Furthermore, the degassing part may separate the cooled water into first water from which dissolved gas is removed and second water containing dissolved gas, and the water supply device may further include a trap part for transferring the second water transferred from the degassing part to the water tank.

Furthermore, the ultrasonic generator may include: a fixing part of which lower surface facing the skin is covered by the water membrane; a receiving part formed between the fixing part and the water membrane, in which the water is received; one or more transducers provided in the fixing part, in which ultrasound is generated; an inlet connected to one side of the receiving part, into which the water supplied from the supply part is injected; and an outlet connected to the other side of the receiving part, in which air or water received in the receiving part is discharged to the supply part, wherein the inlet is connected to an upper side of the receiving part, and wherein the outlet is connected to a circumference of the receiving part.

Furthermore, the system may further include a suction port connected to the water membrane, in which the air received in the receiving part is sucked in; and an opening and closing part for opening and closing the suction port.

Furthermore, a computer program stored in a computer-readable recording medium for executing a method for supplying water to an ultrasonic generator, connected to a computer as hardware may be further provided.

Furthermore, a computer-readable recording medium recording a computer program for executing a method for executing the present disclosure may be further provided.

### [ADVANTAGEOUS EFFECTS OF THE INVENTION]

According to one embodiment of the present invention, it is provided the effect of shortening the procedure preparation time and suppressing the temperature rise of the ultrasonic generator during the procedure.

In addition, according to one embodiment of the present invention, it is provided the effect of improving the degassing and cooling efficiency.

The effects of the present disclosure are not limited to the effects mentioned above, and other effects not mentioned will be clearly understood by those skilled in the art from the description.

### [DESCRIPTION OF THE DRAWINGS]

FIG. 1 is a diagram illustrating a configuration of a water supply system for an ultrasonic generator according to an embodiment of the present disclosure.
FIG. 2 is a water supply flow diagram of a water supply system for an ultrasonic generator according to one embodiment of the present disclosure.
FIG. 3 is a water tank circulation flow diagram of a water supply system for an ultrasonic generator according to one embodiment of the present disclosure.
FIG. 4 is a water membrane circulation flow diagram of a water supply system for an ultrasonic generator according to one embodiment of the present disclosure.
FIG. 5 is a flow diagram of the entire circulation of a water supply system for an ultrasonic generator according to one embodiment of the present disclosure.
FIG. 6 is a water discharge flow diagram of a water supply system for an ultrasonic generator according to one embodiment of the present disclosure.
FIG. 7 is a diagram illustrating a configuration of a water supply system for an ultrasonic generator according to another embodiment of the present disclosure.
FIGS. 8 and 9 are cross-sectional views illustrating an ultrasonic generator of a water supply system for an ultrasonic generator according to one embodiment of the present disclosure.
FIG. 10 is a perspective view illustrating a water membrane of a water supply system for an ultrasonic generator according to one embodiment of the present disclosure.

### [BEST MODE]

In the drawings, the same reference numeral refers to the same element. This disclosure does not describe all elements of embodiments, and general contents in the technical field to which the present disclosure belongs or repeated contents of the embodiments will be omitted. The terms, such as "unit, part, member, and block" may be embodied as hardware or software, and a plurality of "units, parts, members, and blocks" may be implemented as one element, or a unit, a part, a member, or a block may include a plurality of elements.

Throughout this specification, when a part is referred to as being "connected" to another part, this includes "direct connection" and "indirect connection", and the indirect connection may include connection via a wireless communication network. Furthermore, when a certain part "includes" a certain element, other elements are not excluded unless explicitly described otherwise, and other elements may in fact be included.

Furthermore, when a certain part "includes" a certain element, other elements are not excluded unless explicitly described otherwise, and other elements may in fact be included.

In the entire specification of the present disclosure, when any member is located "on" another member, this includes a case in which still another member is present between both members as well as a case in which one member is in contact with another member.

The terms "first," "second," and the like are just to distinguish an element from any other element, and elements are not limited by the terms.

The singular form of the elements may be understood into the plural form unless otherwise specifically stated in the context.

Identification codes in each operation are used not for describing the order of the operations but for convenience of description, and the operations may be implemented differently from the order described unless there is a specific order explicitly described in the context.

Hereinafter, operation principles and embodiments of the present disclosure will be described with reference to the accompanying drawings.

First, High Intensity Focused Ultrasound (HIFU) technology is the latest thermal ablation treatment that burns specific subcutaneous tissues such as tumors in the skin by using the heat generated when high-intensity ultrasound is focused on one point in the skin. This is similar to the principle of focusing warm sunlight with a magnifying glass to light a fire. Since ultrasound easily passes through body tissues, HIFU treatment is performed in a completely non-invasive manner without a knife or even a needle. In other words, by simply pressing the patient's skin on the ultrasound generation surface, specific subcutaneous tissues such as tumors are burned and treated. In addition, HIFU treatment is currently being used to treat uterine fibroids, bone metastases, prostate cancer, breast cancer, pancreatic cancer, liver cancer, and kidney cancer.

This high-intensity focused ultrasound technology may be implemented through an ultrasound generation device. An ultrasound generator can irradiate ultrasound to the surface of a patient's skin.

In this specification, the controller according to an embodiment of the present disclosure in this specification includes various devices that may perform computational processing and provide results to a user. For example, the controller of the ultrasound generating device according to the present disclosure may include a computer, a server device, and a portable terminal, or may be in the form of one of them.

Here, the computer may include, for example, a notebook, a desktop, a laptop, a tablet PC, a slate PC, and the like mounted with a web browser.

The server device is a server that communicates with an external device to process information, and may include an application server, a computing server, a database server, a file server, a mail server, a proxy server, and a web server.

A portable terminal is a wireless communication device that ensures portability and mobility, and may include all kinds of handheld-based wireless communication devices such as PCS (Personal Communication System), GSM (Global System for Mobile communications), PDC (Personal Digital Cellular), PHS (Personal Handyphone System), PDA (Personal Digital Assistant), IMT (International Mobile Telecommunication)-2000, CDMA (Code Division Multiple Access)-2000, W-CDMA (W-Code Division Multiple Access), WiBro (Wireless Broadband Internet) terminal, a smart phone, and the like, and a wearable device such as at least one of a watch, a ring, bracelets, anklets, a necklace, glasses, contact lenses, or a head-mounted device (HMD).

A water supply system for an ultrasonic generator according to an embodiment of the present disclosure may be provided to include an ultrasonic generator including a water membrane for transmitting ultrasonic vibration to a skin; a water supply device configured to supply water to the ultrasonic generator; and a controller configured to selectively control a flow path of the water. Here, the water supply device may include: a supply part for discharging water from the water membrane and supplying the discharged water to the water membrane; a cooling part for cooling the water supplied to the water membrane; a degassing part for removing dissolved gas in the cooled water; and a valve connected to the water membrane, the supply part, the cooling part, and the degassing part. In this case, the controller may control the valve to selectively control a flow path of the water.

The water supply system for an ultrasonic generator may shorten a treatment preparation time and suppress a temperature rise of the ultrasonic generator during the treatment. In addition, the water supply system for an ultrasonic generator may improve a degassing and cooling efficiency.

Hereinafter, the water supply system for an ultrasonic generator will be described in detail.

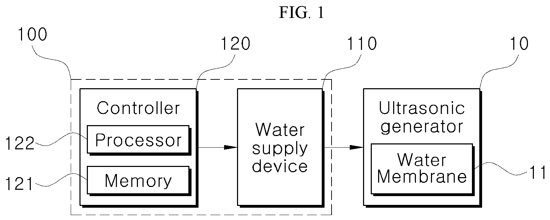

FIG. 1 is a diagram illustrating a configuration of a water supply system for an ultrasonic generator according to an embodiment of the present disclosure.

Referring to FIG. 1, a water supply system 100 for an ultrasonic generator may include a water supply device 110 and a controller 120.

The water supply device 110 may be provided to supply water to an ultrasonic generator 10 including a water membrane 11 that is in close contact with the skin and transmits ultrasonic vibration. In this case, the water membrane 11 may be provided in the transducer of the ultrasonic generator 10, and may be provided so that the water (fluid) from which a dissolved gas is removed is sealed inside, and details thereof will be described below.

The water supply device 110 may include a supply part 111a, 111b, 111c, or 111d, a cooling part 112, a degassing part 113, and a valve 114a, 114b, or 114c.

The supply part 111a, 111b, 111c, or 111d may be provided to discharge water from the water membrane 11 and supply the discharged water to the water membrane 11. In this case, the supply part 111a, 111b, 111c, or 111d may include a discharge pump 111a, a water tank 111b, a supply pump 111c, and a water purification part 111d.

The discharge pump 111a may be provided to pump water discharged from the water membrane 11. For example, the discharge pump 111a may be at least one of a mechanical pump and an electronic pump, and in the case of an electronic pump, it may be provided as the water supply pump and may pump water at a pumping amount determined by the controller 120.

The water tank 111b may be provided to store water pumped by the discharge pump 111a. At this time, the water tank 111b may be provided to be sealable.

The supply pump 111c may be disposed to pump the water discharged from the water tank 111b and supply the water to the water membrane 11. For example, the supply pump 111c may be at least one of a mechanical pump and an electronic pump, and in the case of an electronic pump, may be disposed as the water supply pump and may pump water at a pumping amount determined by the controller 120.

The water purification part 111d may be disposed to purify water pumped by the supply pump 111c. Here, the water purification part 111d may include at least one of a filter or a purifying agent, and may purify water by at least one of the filter or the purifying agent. At this time, the water purification part 111d may be purified under the condition of the injection amount of the purifying agent determined by the controller 120.

The cooling part 112 may be provided to cool the water supplied to the water membrane 11. Here, the cooling part 112 may include a refrigerant and may cool the water by the refrigerant. At this time, the cooling part 112 may be cooled by at least one of the temperature condition and the input condition of the refrigerant determined by the controller 120.

The degassing part 113 may be provided to remove dissolved gas in the cooled water. Here, the degassing part 113 may include a degassing membrane (MDG; Membrane Degassing), and may remove dissolved oxygen in the water by at least one of a degassing membrane-based vacuum decompression degassing method, a heating degassing method, or a deoxygenation treatment method under at least one degassing condition.

The Valve 114a, 114b, or 114c may be provided to be connected to the water membrane 11, the supply part 111a, 111b, 111c, or 111d, the cooling part 112, and the degassing part 113. Here, the valve 114a, 114b, or 114c may include a first valve 114a, a second valve 114b, and a third valve 114c. Here, the first valve 114a, the second valve 114b, and the third valve 114c may be at least one of a mechanical valve and an electronic valve. At this time, the first valve 114a, the second valve 114b, and the third valve 114c may be provided as a 3-port 3-way electronic valve in the case that they are electronic valves. For example, the 3-port 3-way solenoid valve may be a 3-port solenoid valve.

The third valve 114c may be disposed to be connected to the water membrane 11 and the discharge pump 111a. For example, the third valve 114c may be piping-connected to the water membrane 11 and the discharge pump 111a.

The first valve 114a may be disposed to be connected to the third valve 114c, the water tank 111b, and the supply pump 111c. For example, the first valve 114a may be piping-connected to the third valve 114c, the water tank 111b, and the supply pump 111c.

The second valve 114b may be disposed to be connected to the degassing part 113, the water tank 111b, and the water membrane 11. For example, the second valve 114b may be connected to the degassing part 113, the water tank 111b, and the water membrane 11 through pipes.

The controller 120 may be implemented with a memory 121 that stores data on an algorithm for controlling the operation of components within the device or a program that reproduces the algorithm, and at least one processor 122 that performs the operation using the data stored in the memory 121. Here, the memory 121 and the processor 122 may be implemented as separate chips, respectively. In addition, the memory 121 and the processor 122 may be implemented as a single chip.

The memory 121 may store data supporting various functions of the device, programs for the operation of the controller, may store input/output data, a plurality of application programs (or applications) executed on the device, data for the operation of the device, and commands. At least some of these application programs may be downloaded from an external server via wireless communication.

The memory 121 may include at least one type of storage medium among a flash memory type, a hard disk type, an SSD type (Solid State Disk type), an SDD type (Silicon Disk Drive type), a multimedia card micro type, a card type memory (for example, an SD or XD memory, etc.), a random access memory (RAM), a static random access memory (SRAM), a read-only memory (ROM), an electrically erasable programmable read-only memory (EEPROM), a programmable read-only memory (PROM), a magnetic memory, a magnetic disk, or an optical disk. In addition, the memory 122 may be a database that is separate from the device but connected by wire or wirelessly.

The memory 121 may store at least one of data for selectively controlling the flow path of the water, data for controlling the pumping amount of the water, data for controlling the injection amount of the purifying agent, data for controlling at least one of the temperature and injection amount of the refrigerant, or data for controlling the degassing condition. The processor 122 may perform at least one of the operations for selectively controlling the flow path of the water, the operation for controlling the pumping amount of the water, the operation for controlling the injection amount of the purifying agent, the operation for controlling at least one of the temperature and injection amount of the refrigerant, or the operation for controlling the degassing condition.

Here, the processor 122 may control the valve 114a, 114b, or 114c to selectively control the flow path of the water. In this case, the processor 122 may control the first valve 114a, the second valve 114b, and the third valve 114c.

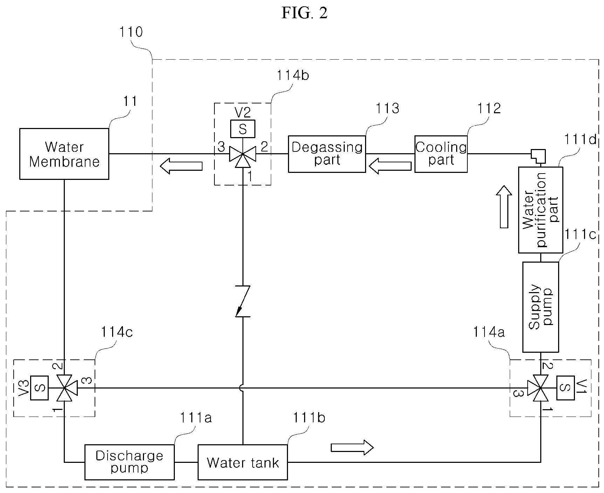

FIG. 2 is a water supply flow diagram of a water supply system for an ultrasonic generator according to one embodiment of the present disclosure.

Referring to FIG. 2, the processor 122 may open the first valve 114a and the second valve 114b and close the third valve 114c to form a water flow path in a direction of the water tank 111b, the supply pump 111c, the water purification part 111d, the cooling part 112, the degassing part 113, and the water membrane 11 of the water supply device 110.

Here, the first valve 114a may cause the water to flow in the direction of the supply pump 111c through the first port and the second port, and the second valve 114b may cause the water to flow in the direction of the water membrane 11 through the second port and the third port.

At this time, the water supply system 100 for an ultrasonic generator according to one embodiment of the present disclosure may operate degassing and cooling from the time of power-on until discharge before the ultrasonic generation of the ultrasonic generator 10 before the procedure.

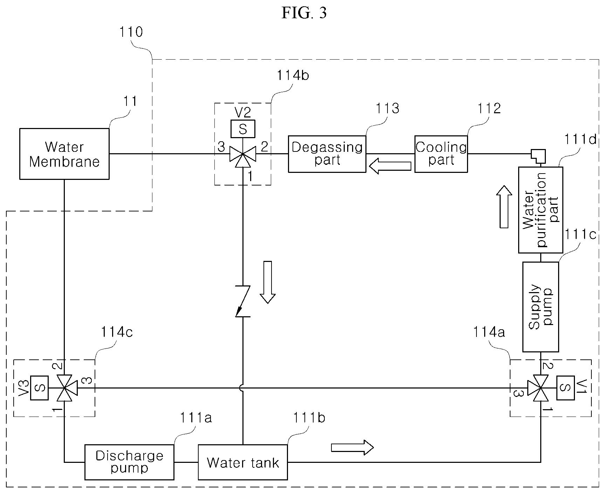

FIG. 3 is a water tank circulation flow diagram of a water supply system for an ultrasonic generator according to one embodiment of the present disclosure.

Referring to FIG. 3, the processor 122 may open the first valve 114a and the second valve 114b and close the third valve 114c to form a water flow path in a direction of the water tank 111b, the supply pump 111c, the water purification part 111d, the cooling part 112, the degassing part 113, and the water tank 111b of the water supply device 110.

Here, the first valve 114a may cause the water to flow in the direction of the supply pump 111c through the first port and the second port, and the second valve 114b may cause the water to flow in the direction of the water tank 111b through the first port and the second port.

At this time, the water supply system 100 for an ultrasonic generator according to one embodiment of the present disclosure may perform primary degassing and cooling by prior to the circulation of the water tank 111b before the water supply.

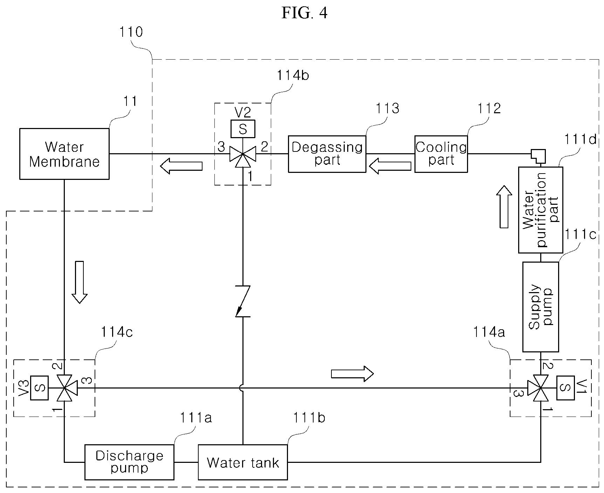

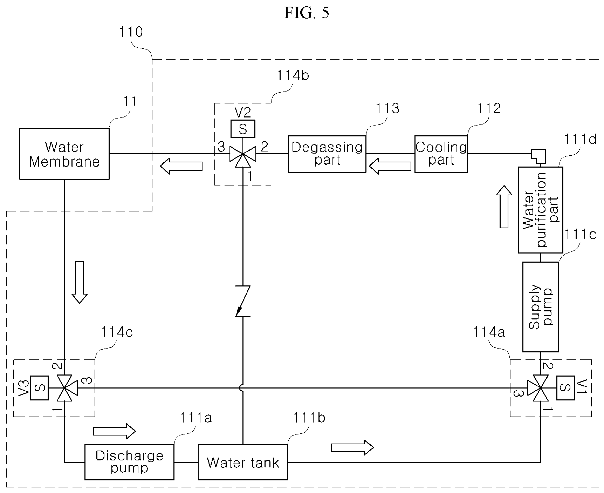

FIG. 4 is a water membrane circulation flow diagram of a water supply system for an ultrasonic generator according to one embodiment of the present disclosure. FIG. 5 is a flow diagram of the entire circulation of a water supply system for an ultrasonic generator according to one embodiment of the present disclosure.

Referring to FIG. 4, the processor 122 may open the first valve 114a, the second valve 114b, and the third valve 114c to form a flow path of water in a direction of the water membrane 11, the supply pump 111c, the water purifier 111d, the cooling part 112, the degassing part 113, and the water membrane 11 of the water supply device 110.

Here, the first valve 114a may allow water to flow in the direction of the supply pump 111c through the first port and the second port, the second valve 114b may allow water to flow in the direction of the water membrane 11 through the second port and the third port, and the third valve 114c may allow water to flow in the direction of the supply pump 111c through the second port and the third port.

Referring to FIG. 5, the processor 122 may open the first valve 114a, the second valve 114b, and the third valve 114c to form a flow path of water in a direction of the water membrane 11, the discharge pump 111a, the water tank 111b, the supply pump 111c, the water purification part 111d, the cooling part 112, the degassing part 113, and the water membrane 11 of the water supply device 110.

Here, the first valve 114a may allow water to flow in the direction of the supply pump 111c through the first port and the second port, the second valve 114b may allow water to flow in the direction of the water membrane 11 through the second port and the third port, and the third valve 114c may allow water to flow in the direction of the discharge pump 111a through the first port and the second port.

**In** this case, the water supply system 100 for an ultrasonic generator according to one embodiment of the present disclosure, when the ultrasonic generator 10 generates ultrasound during the procedure, controls at least one of the preset degassing conditions and the preset refrigerant temperature conditions and input amount conditions, and alternately performs the processes of FIGS. 4 and 5 to reduce the degassing and cooling flow rate.

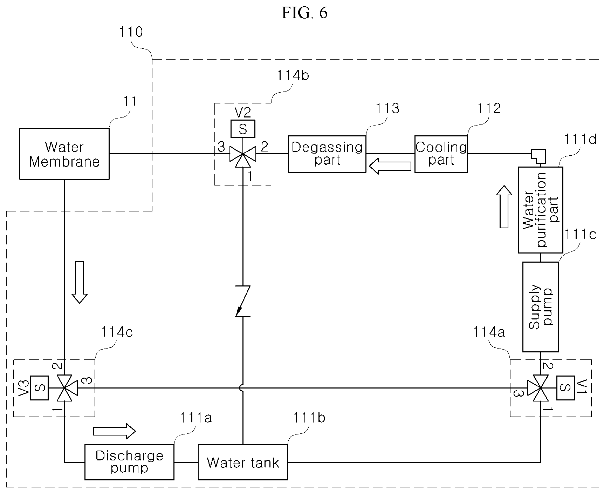

FIG. 6 is a water discharge flow diagram of a water supply system for an ultrasonic generator according to one embodiment of the present disclosure.

Referring to FIG. 6, the processor 122 may close the first valve 114a and the second valve 114b and open the third valve 114c to form a water flow path in a direction of the water membrane 11 and the discharge pump 111a of the water supply device 110.

Here, the third valve 114c may cause the water to flow in the direction of the discharge pump 111a through the first port and the second port.

At this time, the water supply system 100 for an ultrasonic generator according to one embodiment of the present disclosure may sequentially perform the processes of FIGS. 2 to 5 after the process of FIG. 6 to improve the degassing and cooling efficiency.

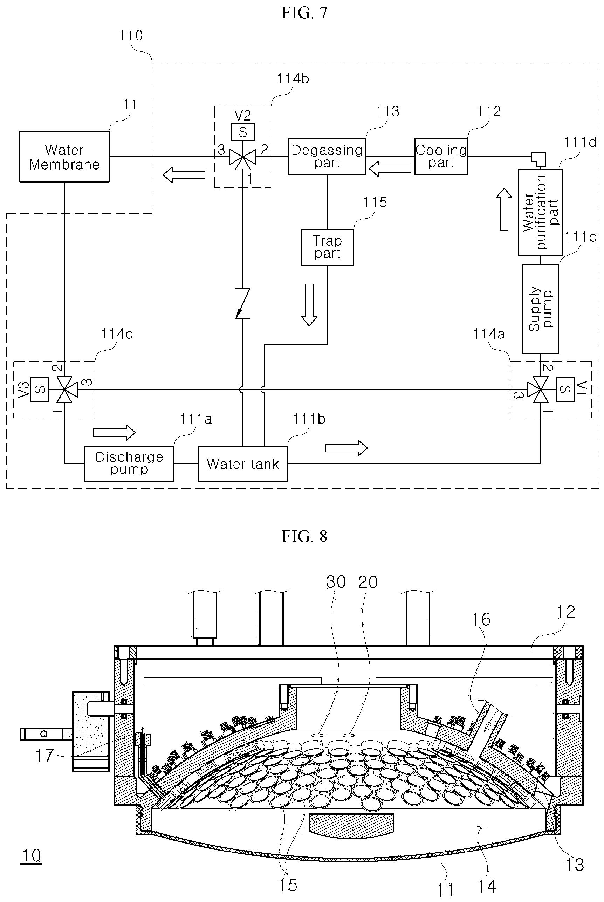

FIG. 7 is a diagram illustrating a configuration of a water supply system for an ultrasonic generator according to another embodiment of the present disclosure.

As shown in FIG. 7, the water supply system for an ultrasonic generator according to another embodiment of the present disclosure may further include a trap part 115.

In this embodiment, the degassing part 113 may separate the cooled water into first water from which dissolved gas has been removed and second water containing dissolved gas. Here, the first water separated by the degassing part 113 may be transferred to the water membrane 11, and the second water may be transferred to the trap part 115 to be described below.

The trap part 115 may transfer the second water transferred from the degassing part 113 to a water tank. For example, the trap part 115 may include a pipe connecting the degassing part 113 and the water tank 111b, and a check valve provided in the pipe to allow the second water to flow from the degassing part 113 to the water tank 111b and to restrict the second water from flowing from the water tank to the degassing part 113.

In the present embodiment, as the first water discharged from the degassing part 113 is transferred to the water membrane 11 and the second water discharged from the degassing part 113 is transferred to the water tank 111b through the trap part 115, the water including the first water and the second water may be circulated in a closed loop along the ultrasonic generator 10 and the water supply device 110. Therefore, in the present embodiment, the total amount of water circulated in a closed loop may be maintained. However, in the case that the total amount of water circulated in the closed loop changes, a leak may have occurred in the ultrasonic generator 10 or the water supply device 110.

The controller 120 calculates the total amount of water circulated in the closed loop along the ultrasonic generator 10 and the water supply device 110, and in the case that the total amount of water changes, the controller 120 may emit an alarm. For example, the alarm may be a warning sound, a warning light, or a combination of a warning sound and a warning light.

As an example, the controller 120 may calculate the total amount of water circulated in the closed loop along the ultrasonic generator 10 and the water supply device 110 based on the measured values of the flow rate of the first water discharged from the deaerator 113 and the flow rate of the second water discharged from the deaerator. Here, the degassing part 113 may be provided with a first flow rate sensor that measures the flow rate of the first water and transmits it to the controller 120, and a second flow rate sensor that measures the flow rate of the second water and transmits it to the controller 120.

As another example, the controller 120 may calculate the total amount of water circulated in a closed loop along the ultrasonic generator 10 and the water supply device 110 based on the value measured for the water level of the water membrane 11. Here, the water level of the water membrane 11 may be measured by a water detection sensor 20 described below.

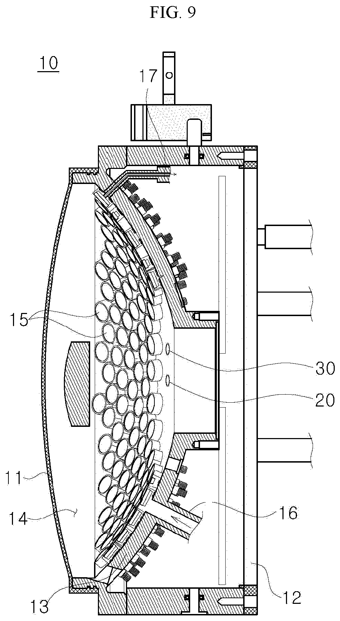

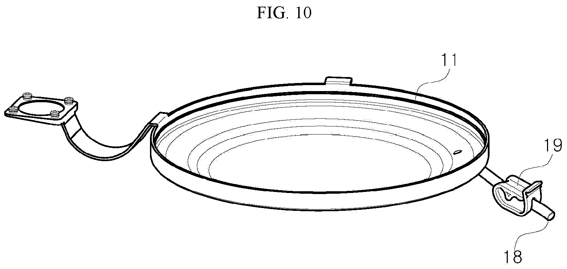

FIGS. 8 and 9 are cross-sectional views illustrating an ultrasonic generator of a water supply system for an ultrasonic generator according to one embodiment of the present disclosure, and FIG. 10 is a perspective view illustrating a water membrane of a water supply system for an ultrasonic generator according to one embodiment of the present disclosure.

As shown in FIG. 8, the ultrasonic generator 10 may include a cartridge housing 12, a fixing part 13, a receiving part 14, a transducer 15, the water membrane 11, an inlet 16, and an outlet 17.

The cartridge housing 12 serves as a basic body of the ultrasonic generator 10. The water membrane 11 and the fixing part 13 may be detachably coupled to the cartridge housing 12.

The fixing part 13 is detachably coupled to the cartridge housing 12 and serves to fix one or more transducers 15. For example, referring to FIG. 8, the fixing part 13 may be detachably coupled to a lower side of the cartridge housing 12.

The lower surface of the fixing part 13 facing the skin may be covered by the water membrane 11. Therefore, the receiving part 14 filled with water from which dissolved gas has been removed may be formed between the fixing part 13 and the water membrane 11.

For example, the fixing part 13 may have a hemispherical shape that protrudes convexly toward the upper side of the housing, and the water membrane 11 may have a hemispherical shape that protrudes convexly toward the lower side of the housing. Therefore, the receiving part 14 formed between the fixing part 13 and the water membrane 11 may have a spherical shape.

In the present embodiment, a water detection sensor 20 that measures the water level of the water filled in the receiving part 14 and transmits it to the controller 120, and a temperature sensor 30 that measures the temperature of the water filled in the receiving part 14 and the temperature of the skin in close contact with the water membrane 11, and transmits them to the controller 120 may be further included. In addition, the controller 120 may further include a display. Here, the display may display the water level transmitted from the water detection sensor 20 to the controller 120, the temperature of the water transmitted from the temperature sensor 30 to the controller, and the temperature of the skin attached to the water membrane 11.

The receiving part 14 is formed between the fixing part 13 and the water membrane 11, and water from which dissolved gas has been removed may be received. For example, the receiving part 14 may be a space formed between the fixing part 13 and the water membrane 11. For another example, the receiving part 14 may be a separate container formed between the fixing part 13 and the water membrane 11.

The transducer 15 is provided in the fixing part 13, and ultrasound may be generated. Here, ultrasound generated from the transducer 15 are transmitted to the water filled in the receiving part 14, thereby generating ultrasonic vibrations in the water filled in the receiving part 14. At this time, ultrasonic vibrations may be generated in the water membrane 11 in conjunction with the ultrasonic vibrations generated in the water.

For example, the transducer 15 may protrude through the fixing part 13 and into the receiving part 14.

For another example, the transducer 15 may be configured in multiples, and the multiple transducers 15 may be arranged radially from the hemispherical fixing part 13 toward the center of the water membrane 11.

The water membrane 11 may be in close contact with the skin and may serve to transmit ultrasonic vibrations to the skin. In this way, when ultrasonic vibration is transmitted to the skin through the water membrane 11, the water filled in the receiving part 14 may cool the transducer 15 and transmit cold air to the skin, thereby preventing excessive temperature rise of the skin.

For example, the water membrane 11 may include an elastic material. Therefore, the water membrane 11 may be deformed into a shape corresponding to the skin while in close contact with the skin.

The inlet 16 is connected to one side of the receiving part 14 so that water supplied from the supply part 111a, 111b, 111c, or 111d may be injected.

The discharge port 17 is connected to the other side of the receiving part 14 so that air or water contained in the receiving part 14 may be discharged. Here, the air contained in the receiving part 14 may be air in the atmosphere that was previously contained in the receiving part 14.

For example, referring to FIG. 8, the inlet 16 may be connected to the upper side of the receiving part 14, and the discharge port 17 may be connected to the periphery of the receiving part 14. Therefore, as shown in FIG. 9, when the ultrasonic generator 10 is rotated so that the inlet 16 is positioned at the top and the discharge port 17 is positioned lower than the inlet 16, and water is injected into the receiving part 14 through the inlet 16, the air contained in the receiving part 14 naturally rises due to the water filled in the receiving part 14 and may be completely discharged through the discharge port 17. As a result, the air contained in the receiving part 14 may be easily discharged through the discharge port 17 without the process of shaking the ultrasonic generator 10 to remove the air contained in the receiving part 14.

As shown in FIG. 10, the ultrasonic generator 10 may further include a suction port 18 and an opening and closing part 19.

The suction port 18 is connected to the water membrane 11, so that air contained in the receiving part 14 may be sucked in. A negative pressure may be formed in the suction port 18 by sucking in the air contained in the receiving part 14, and in this way, the negative pressure formed in the suction port 18 may be formed by a vacuum pump connected to the suction port 18.

The opening and closing part 19 may serve to open/close the suction port 18. For example, the opening and closing part 19 may be a clamp that pressurizes or depressurizes the suction port 18. In another example, the opening and closing part 19 may be a stopper. In one embodiment of the present disclosure, prior to the procedure, the circulation of the water tank 111b is performed before the ultrasonic generation of the ultrasonic generator 10, and during the procedure, the circulation of the water membrane 11 is performed continuously when the ultrasonic generator 10 generates ultrasound, thereby shortening the procedure preparation time and suppressing the temperature rise of the ultrasonic generator 10 during the procedure.

In one embodiment of the present disclosure, by changing the previous open water tank to a sealed water tank, the degassing and cooling deviations in the processes of FIGS. 3 to 5 may be minimized. At least one component may be added or deleted in accordance with the performance of the components illustrated in FIGS. 1 to 10. In addition, it will be readily understood by those skilled in the art that the mutual positions of the components may be changed in accordance with the performance or structure of the system.

Meanwhile, the disclosed embodiments may be implemented in the form of a recording medium storing instructions executable by a computer. The instructions may be stored in the form of program codes, and when executed by a processor, may generate a program part to perform the operations of the disclosed embodiments. The recording medium may be implemented as a computer-readable recording medium.

The computer-readable recording medium includes all types of recording media storing instructions that may be deciphered by a computer. For example, there may be a ROM (Read Only Memory), a RAM (Random Access Memory), a magnetic tape, a magnetic disk, a flash memory, an optical data storage device, and the like.

The disclosed embodiments have been described with reference to the attached drawings as described above. A person skilled in the art to which the present disclosure pertains will understand that the present disclosure may be implemented in a different form from the disclosed embodiments without changing the technical idea or essential features of the present disclosure. The disclosed embodiments are illustrative and should not be construed as limiting.

## Claims

1. A water supply system for an ultrasonic generator, comprising:
an ultrasonic generator configured to irradiate a patient's skin with ultrasonic waves, wherein the ultrasonic generator includes a water membrane;
a water supply device configured to supply water to the ultrasonic generator; and
a controller configured to selectively control a flow path of the water.

2. The system according to claim 1, wherein the water supply device includes:
a supply part configured to discharge water from the water membrane and supply the discharged water to the water membrane;
a cooling part configured to cool the water supplied to the water membrane;
a degassing part configured to remove dissolved gas in the cooled water; and
one or more valves connected to the water membrane, the supply part, the cooling part, and the degassing part.

3. The system according to claim 2, wherein the one or more valves include a first valve, a second valve, and a third valve;
wherein the third valve is connected to the water membrane and the supply part;
wherein the first valve is connected to the third valve and the supply part; and
wherein the second valve is connected to the degassing part, the supply part, and the water membrane.

4. The system according to claim 2, wherein the supply part includes:
a discharge pump configured to pump water discharged from the water membrane;
a water tank configured to store the water pumped by the discharge pump;
a supply pump configured to pump the water discharged from the water tank and supply the water to the water membrane; and
a water purification part configured to purify the water pumped by the supply pump.

5. The system according to claim 4, wherein the controller is configured to:
open the first valve and the second valve and close the third valve to form a flow path of the water in a direction of the water tank, the supply pump, the water purification part, the cooling part, the degassing part, and the water membrane.

6. The system according to claim 4, wherein the controller is configured to:
open the first valve and the second valve and close the third valve to form a flow path of the water in a direction of the water tank, the supply pump, the water purification part, the cooling part, the degassing part, and the water tank.

7. The system according to claim 4, wherein the controller is configured to:
open the first valve, the second valve, and the third valve to form a flow path of the water in a direction of the water membrane, the supply pump, the water purification part, the cooling part, the degassing part, and the water membrane.

8. The system according to claim 4, wherein the controller is configured to:
open the first valve, the second valve, and the third valve to form a flow path of the water in a direction of the water membrane, the discharge pump, the water tank, the supply pump, the water purifier, the cooling part, the degassing part, and the water membrane.

9. The system according to claim 4, wherein the controller is configured to:
close the first valve and the second valve, and open the third valve to form a flow path of the water in a direction of the water membrane and the discharge pump.

10. The system according to claim 4, wherein the degassing part is configured to separate the cooled water into first water from which dissolved gas is removed and second water containing dissolved gas; and
wherein the water supply device further includes a trap part for transferring the second water transferred from the degassing part to the water tank.

11. The system according to claim 2, wherein the ultrasonic generator includes:
a fixing part of which a lower surface facing the patient's skin is covered by the water membrane;
a receiving part formed between the fixing part and the water membrane, in which the water is received;
one or more transducers provided in the fixing part, wherein the one or more transducers are configured to generate the ultrasonic waves;
an inlet connected to one side of the receiving part, into which the water supplied from the supply part is injected; and
an outlet connected to the other side of the receiving part, in which air or water received in the receiving part is discharged to the supply part;
wherein the inlet is connected to an upper side of the receiving part, and
wherein the outlet is connected to a circumference of the receiving part.

12. The system according to claim 11, further comprising:
a suction port connected to the water membrane, in which the air received in the receiving part is sucked in; and
an opening and closing part configured to open and close the suction port.
